# EUROPEAN PATENT SPECIFICATION

(11) **EP 3 773 595 B1**
(45) Date of publication and mention of the grant of the patent: **12.07.2023**
(21) Application number: 19721103.0
(22) Date of filing: 28.03.2019
(51) Int. Cl.: A61K 31/5377, A61P 35/00, A61K 39/395

(54) **ANTICANCER PHARMACEUTICAL COMPOSITIONS FOR COMBINED THERAPY**
PHARMAZEUTISCHE ZUSAMMENSETZUNGEN GEGEN KREBS ZUR KOMBINIERTEN THERAPIE
COMPOSITIONS PHARMACEUTIQUES ANTICANCÉREUSES POUR THÉRAPIE COMBINÉE

(30) Priority: 29.03.2018 IT 201800004082
(43) Date of publication of application: 17.02.2021
(73) Proprietor: Berlin-Chemie AG, 12489 Berlin (DE)
(72) Inventor: MERLINO, Giuseppe, 00071 Pomezia RM (IT); BIGIONI, Mario, 00071 Pomezia RM (IT); BINASCHI, Monica, 00071 Pomezia RM (IT); PELLACANI, Andrea, 50131 Firenze (IT)
(74) Representative: Di Giovine, Paolo
(86) International application number: PCT/IB2019/052541
(87) International publication number: WO 2019/186451

(56) References cited:
- EP-A2- 2 949 326
- WO-A1-2012/118978
- WO-A1-2015/095807
- WO-A1-2016/142508
- US-A1- 2013 123 255
- TANAKA H ET AL: "122 Novel class I PI3K inhibitor CH5132799: potential clinical application in rational combination with molecular targeted therapeutics", EUROPEAN JOURNAL OF CANCER. SUPPLEMENT, PERGAMON, OXFORD, GB, vol. 8, no. 7, 1 November 2010 (2010-11-01), page 45, XP027497810, ISSN: 1359-6349, DOI: 10.1016/S1359-6349(10)71827-9 [retrieved on 2010-11-01]

## Description

### Technical field of the invention

The present invention relates to new combinations of antitumour or anticancer agents for the tumour combination therapy, pharmaceutical compositions intended for combined use and kit containing compositions of different antitumour agents for combined use. In particular the present invention relates to combinations of various tumour agents with the PI3K-Class I inhibitor 5-(7-methanesulfonyl-2-morpholin-4-yl-6,7-dihydro-5H-pyrrolo[2,3-d]pyrimidin-4-yl)-pyrimidin-2-il-amine or pharmaceutically acceptable salts thereof.

### State of prior art

The enzyme phosphatidylinositol-3-kinase (PI3K) is known as type of phosphorylase that phosphorylates in position 3 of an inositol ring of the phosphatidylinositol and it is expressed widely in the whole body. PI3K is known to be activated by stimulations from growth factors, hormones and the like. PI3K activates Akt and PDK1 and it is involved in survival signals which inhibit the cell death, regulate the cytoskeleton, the glucose metabolism, the vesicle transport and the like.

PI3K is classified into three groups, precisely Class I, Class II and Class III based upon the primary structure, the type of phosphatidylinositols which act as substrate. The enzymes of class I are sub-classified into class Ia and Ib depending upon the activation mechanism. The class Ia includes the sub-types p110α, p110β and p110δ, and each one forms a heterodimer complex with an adjusting sub-unity (p85) and it is activated by a tyrosine-kinase receptor and the like. The class 1b includes a sub-type p110γ activated by the sub-unity βγ (Gβγ) of a protein G of trimer and forms a heterodimer with an adjusting sub-unity (p101).

Recently, three phenomena have been reported in several types of cancers (and in particular the ovarian cancer, the colon cancer and the breast cancer): an amplification of the PIK3CA p110α coding gene, the constitutive activation due to mutation and high expression of p110α at protein level. Consequently, the apoptosis inhibition by means of constitutive activation of the survival signals is considered partially responsible for the tumorigenesis mechanism. In fact, it was reported that the PIK3CA is involved as oncogene in the ovarian cancer (Natura Genet. 21, 99-102, (1999); that the frequency of the PIK3CA gene mutations in the human cancers is high (Science, 304, 554, (2004) and that there is an increase in the levels of activity of phosphoinositol 3-kinase in the colorectal cancers (Cancer, 83, 41-47 (1998)).

Based upon this knowledge, it was found that inhibitors of PI3K and specifically of the p110α activity exert an antitumour action, in particular in the cancers with high PI3K activity.

Thereamong the compound 5-(7-methanesulfonyl-2-morpholin-4-yl-6,7-dihydro-5H-pyrrolo[2,3-d]pyrimidin-4-yl)-pyrimidin-2-amine, also known in literature as CH5132799, resulted to be particularly effective as antitumour agent having inhibitory activity on PI3K, having the following formula I

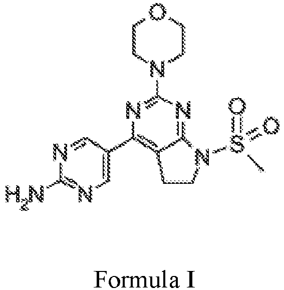

The compound and method of its preparation were described in WO-A-2008/018426. The pharmacological features of the compound were described in scientific literature by Jun Ohwada et al. on Bioorganic & Medicinal Chemistry Letter, 21 (2001) pages 1767-1772. Moreover, the association of the compound of formula I with the monoclonal antibody trastuzumab in the treatment of cancer forms resistant to the latter is described by Hiroshi Tanaka et al. on Clin. Cancer Res. 17, (10) May 15 (2011).

TANAKA H ET AL: "122 Novel class I PI3K inhibitor CH5132799: potential clinical application in rational combination with molecular targeted therapeutics",EUROPEAN JOURNAL OF CANCER. SUPPLEMENT, PERGAMON, OXFORD, GB, vol. 8, no. 7, 1 November 2010 (2010-11-01), page 45,
discloses the activity of the combination of CH5132799 (the present PI3K inhibitor) with trastuzumab (anti-HER2 antibody) on tumor xenografts, namely on trastuzumab-insensitive breast cancer cell line KPL-4, which harbors Her2 amplification and PIK3CA mutation (H1047R).

Although the PI3K inhibitors are very effective, however the problem remains that not all the cancer forms respond effectively to a same medicament even when this is associated to other active principles.

The object of the present invention then is to provide a medicament free from such drawbacks.

Therefore, the object of the present invention is to make available new antitumour combinations providing the co-therapy with the compound of formula I and an additional antitumour agent capable of strengthening the final therapeutic effectiveness Said object is obtained with a composition the main features thereof are specified in the first claim, whereas other features are specified in the remaining claims.

### Abstract of the invention

The present invention is based upon the finding by the present inventors that several known antitumour compounds are capable of improving the effectiveness of the compound of formula I, through a synergic effect, thus allowing to treat effectively (or at least more effectively) types of cancers resistant to the previous treatments.

The invention is defined by the claims. Any subject-matter falling outside the scope of the claims is provided for information purposes only.

Therefore, the present invention relates to the following embodiments:
Pharmaceutical compositions containing as active compound the PI3K-Class I inhibitor compound: 5-(7-methanesulfonyl-2-morpholin-4-yl-6,7-dihydro-5H-pyrrolo[2,3-d]pyrimidin-4-yl)-pyrimidin-2-amine or the pharmaceutically acceptable salt thereof, for use in an anticancer treatment in association with a second anticancer compound as defined in the claims, namely cetuximab, bevacizumab, fulvestrant, or gefitinib.

Pharmaceutical compounds wherein said second anticancer compound is selected from the group comprising an antibody such as an anti-HER2 antibody different from trastuzumab, an EGF anti-receptor antibody, an antibody that inhibits the vascular endothelial growth factor-A (VEGF-A), or an epidermal growth factor receptor (EGFR) inhibitor, a selective degrader/modulator of the estrogen receptor (ER) or a protein kinase inhibitors are presently disclosed but are not part of the invention.

Part of the invention are pharmaceutical compounds wherein said second anticancer compound is selected from the group comprising an EGF anti-receptor antibody, namely the antibody cetuximab, an antibody that inhibits the vascular endothelial growth factor-A (VEGF-A), namely the antibody bevacizumab, or an epidermal growth factor receptor (EGFR) inhibitor, namely the compound gefitinib, a selective degrader/modulator of the estrogen receptor (ER), namely fulvestrant for treating cancer.

The following is also part of the invention.

Pharmaceutical compositions according to the invention for use in an anticancer treatment of colon cancer, prostate cancer, breast cancer, lung cancer, ovarian cancer.

Pharmaceutical compositions suitable for the oral administration of the PI3k-Class I inhibitor, for use in association with a composition containing the second anticancer compound for use in treating cancer.

Pharmaceutical compositions for use in association with a pharmaceutical composition suitable for the oral or parental administration of the second anticancer compound for use in treating cancer.

The present invention further relates to:
Kit of parts comprising a pharmaceutical composition containing as active compound the PI3K-Class I inhibitor compound: 5-(7-methanesulfonyl-2-morpholin-4-yl-6,7-dihydro-5H-pyrrolo[2,3-d]pyrimidin-4-yl)-pyrimidin-2-amine or the pharmaceutically acceptable salt thereof, and a second pharmaceutical composition containing a second anticancer compound, as defined in the claims, namely cetuximab, bevacizumab, fulvestrant, or gefitinib, for use in treating cancer.

Kit of parts, wherein said second anticancer compound is selected from the group comprising an antibody such as an anti-HER2 antibody different from trastuzumab, an EGF anti-receptor antibody, preferably the antibody cetuximab, an antibody that inhibits the vascular endothelial growth factor-A (VEGF-A), preferably the antibody bevacizumab, a selective degrader/modulator of the estrogen receptor (ER), preferably the compound fulvestrant, or an epidermal growth factor receptor (EGFR) inhibitor, preferably the compound gefitinib, or a protein kinase inhibitor are disclosed but are not part of the invention.

Object of the invention are also the following.

Kit of parts according to any one of claims 13 to 14 wherein the anticancer combination treatment is a treatment of colon cancer, prostate cancer, breast cancer, lung cancer, ovarian cancer.

Kit of parts according to the invention wherein the first composition is suitable for the oral administration of the PI3k-Class I inhibitor, and the second composition is suitable for the oral or parental administration for treating cancer.

The advantages offered by the invention can be referred to the increased effectiveness even in the treatment of forms of cancer or tumour resistant to preceding treatments and recidivisms.

### Brief description of figures

Figure 1: Shows the antitumour activity of the compound MEN1611 (that is the compound of Formula I) in combination with trastuzumab on breast cancer cells JIMT-1 (HER2+, mutated on the PIK3CA gene) α) transplanted in nude mouse (immunodeficient).

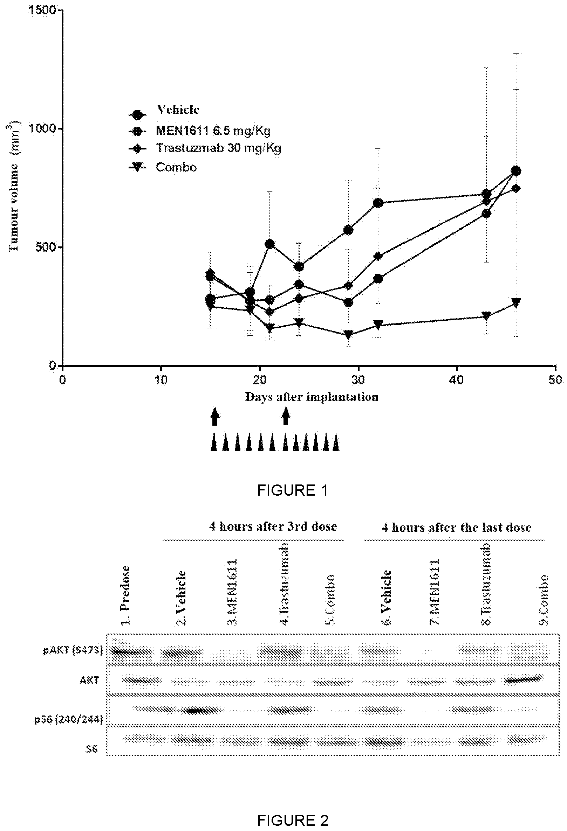

Figure 2: the antitumour activity of MEN1611 is supported even by its pharmacodynamic activity, by means of the assay of immunoblotting, wherein the phosphorylation of two proteins AKT and S6, proteins downstream of PI3K, was evaluated. The treatment with the MEN1611 alone or in combination with trastuzumab (Combo), of JIMT-1, significantly inhibits the phosphorylation of the two proteins AKT and S6 thus demonstrating an action of MEN1611 on such pathway. The phosphorylation instead is not inhibited after the treatment with trastuzumab. The analysed tumour masses were collected 4 hours after the third and last dose of MEN1611.
Figure 3: Shows the antitumour activity of the compound MEN1611 in combination with trastuzumab on breast cancer cells HCC1954 (HER2+, mutated on the PIK3CA gene) transplanted in nude mouse (immunodeficient).

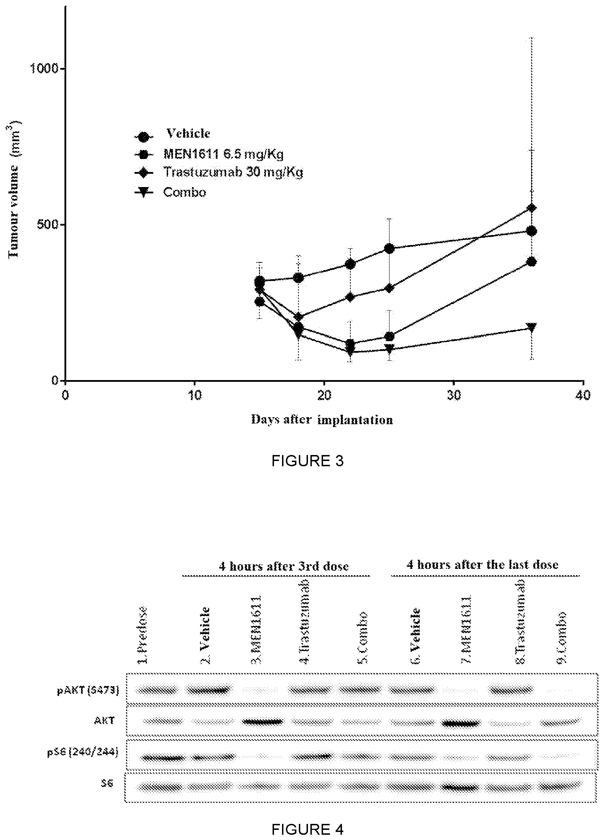

Figure 4: The antitumour activity of MEN1611 is supported even by its pharmacodynamic activity, by means of the assay of immunoblotting, wherein the phosphorylation of two proteins AKT and S6, proteins downstream of PI3K, was evaluated. The treatment with MEN1611 alone or in combination with trastuzumab (Combo), of HCC1954, significantly inhibits the phosphorylation of the two proteins AKT and S6 thus demonstrating an action of MEN1611 on such pathway. The phosphorylation on the contrary is not inhibited after the treatment with trastuzumab. The analysed tumour masses were collected 4 hours after the third and last dose of MEN1611.
Figure 5: Shows the antitumour activity of the compound MEN 1611 in combination with cetuximab on cancer cells HCC70 (triple negative breast cancer, wild type for the PIK3CA gene and with loss of the PTEN gene) transplanted in nude mouse (immunodeficient)

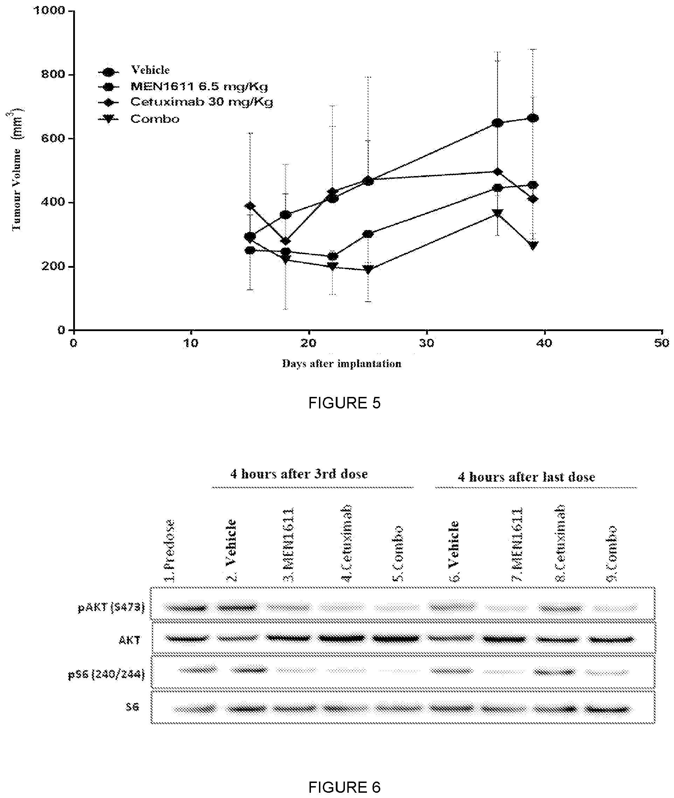

Figure 6: the antitumour activity of MEN1611 is supported even by its pharmacodynamic activity, by means of the assay of immunoblotting, wherein the phosphorylation of two proteins AKT and S6, proteins downstream of PI3K, was evaluated. The treatment with MEN1611 alone or in combination with cetuximab (Combo), of HCC70, significantly inhibits the phosphorylation of the two proteins AKT and S6 thus demonstrating an action of MEN1611 on such pathway. The phosphorylation on the contrary is not inhibited after the treatment with cetuximab. The analysed tumour masses were collected 4 hours after the third and last dose of MEN1611.
Figure 7: Shows the antitumour activity of the compound MEN1611 in combination with cetuximab on RKO cancer cells (colorectal cancer, mutated on the PIK3CA gene and mutated on the BRAF gene) transplanted in nude mouse (immunodeficient).

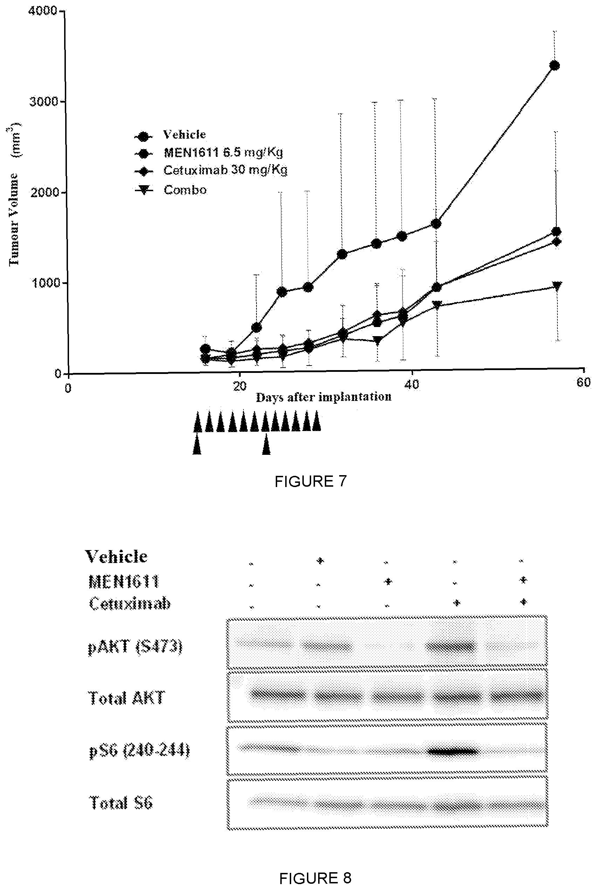

Figure 8: the antitumour activity of MEN1611 is supported even by its pharmacodynamic activity, by means of the assay of immunoblotting, wherein the phosphorylation of two proteins AKT and S6, proteins downstream of PI3K, was evaluated. The treatment with MEN1611 alone or in combination with cetuximab (Combo), of RKO, significantly inhibits the phosphorylation of the two proteins AKT and S6 thus demonstrating an action of MEN1611 on such pathway. The phosphorylation on the contrary is not inhibited after the treatment with cetuximab. The analysed tumour masses were collected 4 hours after the third and last dose of MEN1611.
Figure 9: Shows the antitumour activity of the compound MEN1611 in combination with cetuximab on cancer cells HT-29 (colorectal cancer, mutated on the PIK3CA gene and mutated on the BRAF gene) transplanted in nude mouse (immunodeficient).

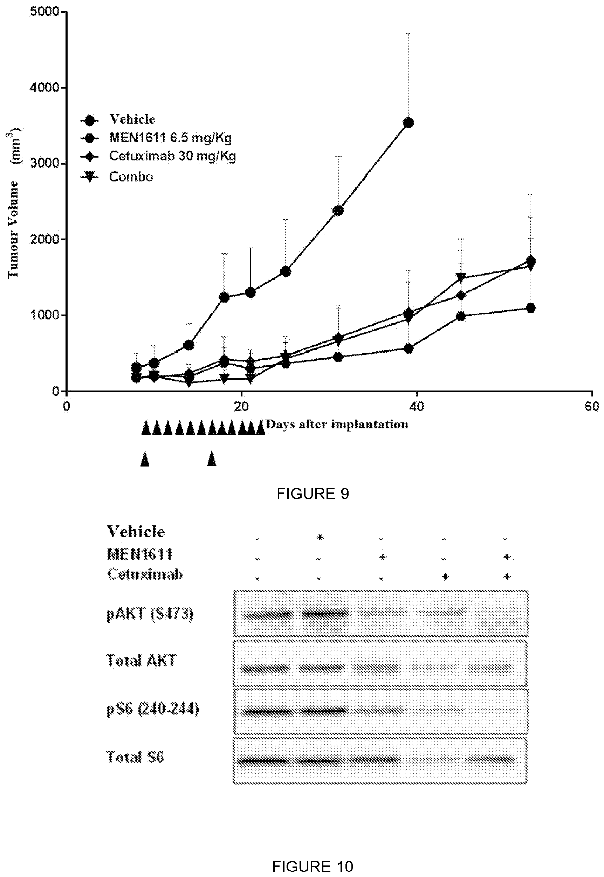

Figure 10 the antitumour activity of MEN1611 is supported even by its pharmacodynamic activity, by means of the assay of immunoblotting, wherein the phosphorylation of two proteins AKT e S6, proteins downstream of PI3K, was evaluated. The treatment with MEN1611 alone or in combination with cetuximab (Combo), of HT-29, significantly inhibits the phosphorylation of the two proteins AKT and S6 thus demonstrating an action of MEN1611 on such pathway. The phosphorylation on the contrary is not inhibited after the treatment with cetuximab. The analysed tumour masses were collected 4 hours after the third and last dose of MEN1611.
Figure 11: Shows the antitumour activity of the compound MEN1611 in combination with bevacizumab on tumour cells of colorectal cancer, mutated on the PIK3CA gene and on the KRAS gene, transplanted in nude mouse (immunodeficient), model CTG-1509.
Figure 12: Shows the antitumour activity of the compound MEN1611 in combination with erlotinib on cancer cells NCI-H-1975 (non-small cell lung cancer, mutated on the PIK3CA gene and on the EGFR gene) transplanted in nude mouse (immunodeficient).

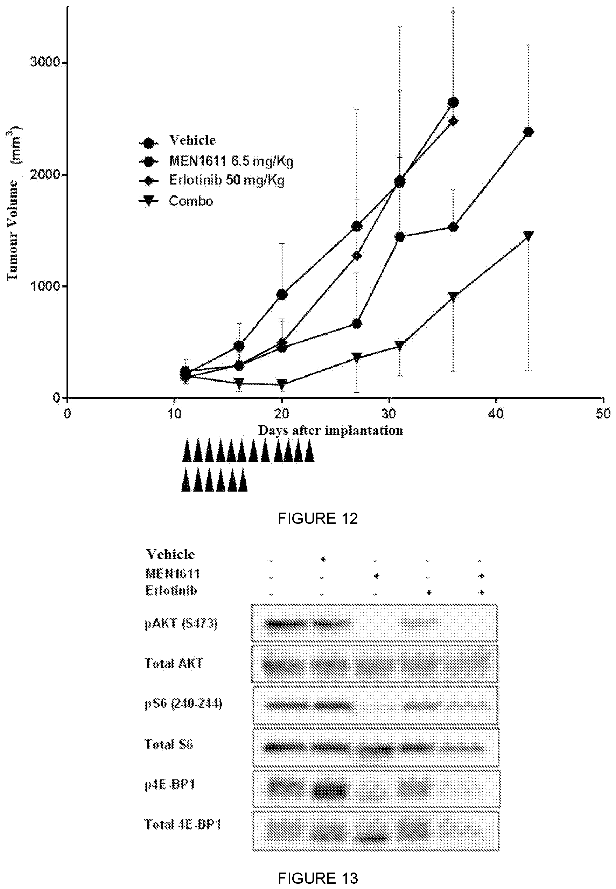

Figure 13: the antitumour activity of MEN1611 is supported even by its pharmacodynamic activity, by means of the assay of immunoblotting, wherein the phosphorylation of two proteins AKT and S6, proteins downstream of PI3K, was evaluated. The treatment with MEN1611 alone or in combination with erlotinib (Combo), of NCI-H-1975, significantly inhibits the phosphorylation of the two proteins AKT and S6 thus demonstrating an action of MEN1611 on such pathway. The phosphorylation on the contrary is not inhibited after the treatment with erlotinib. The analysed tumour masses were collected 4 hours after the third and last dose of MEN1611.
Figure 14: Shows the antitumour activity of the compound MEN1611 in combination with erlotinib on cancer cells NC-H-292 (non-small cell lung cancer, wild type for the PIK3CA and EGFR gene) transplanted in nude mouse (immunodeficient).
Figure 15: shows the synergistic effect of the combination of MEN1611 with Fulvestrant (degrader/modulator of the estrogen receptor) on the T47D cell line of breast cancer HER2+/ER+, mutated on the PIK3CA gene.
Figure 16: Shows the antitumour activity of the compound MEN1611 in combination with erlotinib on cancer cells RU278 (non-small cell lung cancer, mutated on the PIK3CA gene e EGFR) transplanted in nude mouse (immunodeficiencies).
Figure 17: Shows the antitumour activity of the compound MEN1611 in combination with gefitinib on cancer cells RB1 (non-small cell lung cancer, wild type for the PIK3CA gene and mutated on the EGFR gene) transplanted in nude mouse (immunodeficiencies).

### DETAILED DESCRIPTION

The PI3K-Class-I inhibitor compound *5-(7-methanesulfonyl-2-morpholin-4-yl-6, 7-dihydro-5H-pyrrolo[2,3-d]pyrimidin-4-yl)pyrimidin-2-il-amine* (even reported as CH5132799 or MEN1611) or pharmaceutically acceptable salts thereof is described in WO2008/018426. In particular the preparation of the compound is described in the example 1-D-02 thereto one refers. The compound itself and the pharmacological characterization is described in scientific literature by Jun Ohwada et al. (2011) (above) or Hiroshi Tanaka et al. (2011) (above).

By taking into consideration the chemical formula of the inhibitor of formula I, under pharmaceutically acceptable salt an acid or basic salt is meant.

Examples of acid salts include hydrochlorides, (hydrobromides), (hydroiodides), nitrates, sulphates, bisulphates, phosphates, acid phosphates, acetates, lactates, citrates, acid citrates, tartrates, bitartrates, succinates, oxalates, malates, fumarates, gluconates, malonates, saccharates, benzoates, mandelates, salicylates, trifluoroacetates, propionates, glutarates, methane-sulphonates, ethane-sulphonates, benzensulphonates, p-toluensulphonate and 1,1'-methylene-bis-2-hydroxy-3-naphtates.

Examples of basic salts include salts of alkali metals such as sodium salts and potassium salts, salts of alkaline-earth metals such as calcium salts and magnesium salts, ammonium salts, addition salts with water-soluble amines such as salts of N-methylglucamine, inferior alkanol ammonium salts and salts derived from other pharmaceutically acceptable bases of organic amines.

The compounds having antitumour activity for use in a combination therapy with the compound of formula I are all known medicaments, classified in the class L01 (antineoplastic agents) or WHO Collaborative Centre (WHOCC) ATC/DDD Index.

### Pharmaceutical compositions

Considering the discrepancy between the effective dosages of the different antitumour agents and even between the dosage regimes, the two antitumour agents to be administered as combination will be formulated in autonomous compositions.

The pharmaceutical compositions of the present invention can be formulated and administered by oral or parenteral route (such as by intravenous, intramuscular, subcutaneous, rectal, nasal, intracisternal, vaginal, abdominal, intracystic route or locally). Examples of preparations for the oral administration include tablets, capsules, granules, powders, pills, solutions and aqueous and non-aqueous oral suspensions. Examples of preparations for the parental administration include aqueous or oily solution for intravenous, intramuscular, subcutaneous injections. Other formulations such as ointments, gels, creams, suppositories, sprays by oral or nasal route, emulsions, oily agents and suspending agents, can be equally used if suitable to the contingent situation. The solutions for parental use usually are distributed in containers suitable for the administration in small individual doses. However, considering that the dosage is proportioned to the body weight, the formulation of the various active principles should be so as to allow the administration of customized quantities of the medicament and not the administration of standard quantities. Moreover, the administration form can be adapted to the various administration methods including controlled release formulation in the sub-cutaneous transplant mode.

In particular the compound di formula I, that is the PI3K inhibitor, usually is administered by oral route and therefore it will be formulated in any form suitable for such administration route. However, other administration routes are not excluded.

The second compound can be administered depending upon its nature by parental or oral route and therefore it will be suitably formulated.

The dosages of the PI3K inhibitor as well as of the second antitumour agent can be suitably modified based upon symptoms, age, body weight, relative health state, presence of other drugs, administration route and the like.

The typical dosage of PI3K inhibitor of formula I effective for a patient in case of oral preparation preferably is from 0.1 to 1000 mg and more preferably from 1 to 100 mg, per kg of body weight daily. Preferred dosages are from 1 to 10 mg/Kg for example 3, 4, 5, 6, 7, 8 or 9 mg/Kg body weight.

In particular, daily dosages from 40 mg/die to 100 mg/die are administered, for example two 48-mg capsules daily (total 96 mg daily).

In case of parental administration, the typical effective quantity preferably is from 0.1 to 1000 mg and more preferably from 1 to 100 mg per kg of body weight daily.

The second medicament, which is a medicament already on the market, is administered in dosages complying with the prescriptions given in the respective technical reports shown with the application for the Marketing Authorization (MA). These dosages usually vary from 1 to 100 mg/Kg body weight, or from 10 to 80 mg/Kg or from 20 to 70 mg/Kg or from 30 to 50 mg/Kg body weight.

Hereinafter the dosages prescribed for clinical use of the following medicaments are shown (only the compounds claimed being part of the invention):
Cetuximab: The dosage is both for monotherapy and for combination: initial dose: 400 mg/m² IV for 2 hours; maintenance: 250 mg/m² IV for 60 min qWeek until disease progression or toxicity events
Trastuzumab: the therapeutic scheme is very complex, the doses are from 2 mg/Kg to 8 mg/Kg IV.
Bevacizumab: 5-10 mg/kg IV q2Weeks
Eriotinib: as it is a small molecule, it is similar to MEN1611, the dosage is 150 mg daily for oral route, until the patient progresses or has toxicity events
Fulvestrant: the dosage both for monotherapy and for combinations is: 500 mg intramuscular on the days 1, 15, 29 and then once a month
Gefitinib: the dosage for monotherapy is 250 mg daily for oral route, until the patient progresses or has toxicity events.

Even the dosage regimes are devised to obtain the maximum effectiveness of each single antitumour agent used in the combinations of the invention. Therefore, the co-therapy with the combinations of the invention does not involve necessarily that the administrations are performed simultaneously, but simply that the treatments are overlapped. Therefore, the administration of the two active principles could be both contemporary and in quick sequence, for example separated by few minutes, or hours, as well as alternated and separated by a longer period, for example days or weeks.

For example the PI3K inhibitor can be administered once a day for 7, 10, 12, 15 or 20 days (qldx7, 10, 12, 15 o 20).

The second medicament can be administered according to an identical or different regime, but on alternate days with respect to the first medicament.

Differently, when the second medicament is an antibody this can be administered at intervals of several days, for example every 5 days, once a week, once every two weeks or once a month.

Examples of cancer treated with the combinations of the present invention include solid tumours, whereas examples of solid tumours include breast cancer, colon cancer, colorectal cancer, ovarian cancer, prostate cancer and non-small cell lung cancer.

An additional embodiment of the invention consists in a kit of parts, that is a package ready for use containing in a first section the first medicament that is the PI3K inhibitor of formula I, in a second section the second medicament and a package leaflet with instructions for the combined, contemporary, consecutive or alternated administration of the two medicaments. The kit comprises a number of mono-doses of the first and the second medicament required and sufficient for a complete treatment cycle.

In particular the first section will include a blister of mono-doses of the first medicament for oral administration, for example tablets, stiff or soft capsules or phials of lyophilised medicament or powder sachets or granulate sufficient for a complete cycle, whereas the second section will equally include blisters or phials or sachets, as in the first section, including the second medicament for oral use or phials including the solution or phials including the lyophilizate of the second medicament for parental use. In case of lyophilised formulations of the first or the second medicament the respective sections will include the required disposable amounts of the solvent suitable to bring the lyophilizate back to solution.

The number of mono-doses of the first and of the second medicament will correspond to the dose required for a complete cycle according to what prescribed.

For example the first section will include a number of unitary doses (tablets, capsules, etc) sufficient for the daily administration for a period ranging from 7 to 40 days or from 10 to 30 days or from 12 to 20 days (q1dx7-40 or q1dx10-30 or q1dx12-20).

The second section will include unitary doses sufficient for one administration or daily or weekly or bi-weekly or monthly administration for a period, according to prescription, overlapped to the period of treatment with the first medicament. For example by starting the two overlapped treatments on the same day 1, the second medicament could be subsequently administered every 4, 5, 7, 10 or 12 days. When the second medicament is an antibody usually a limited number (2-4) of administrations are sufficient for a complete cycle.

Other obvious embodiments providing combined treatments of the PI3K inhibitor with a second medicament are included within the object of the present invention.

The invention will be illustrated hereinafter in the experimental section and in the embodiment examples intended by purely way of illustration only.

### Experimental section

MEN1611 is obtained by MENARINI RICERCHE SPA Pisa. The powder was dissolved in a storage solution constituted by DMSO/Cremophor EL (volumetric 50%/50%), aliquoted in the quantity to be used daily and all aliquots are stored in refrigerator at 4°C until the day of their use. The storage solution del MEN1611 is prepared at a concentration of 6.5 mg/ml. The diluent solution was prepared by dissolving hydroxypropyl-beta cyclodextrin (HPCD) and polyethylene glycol 400 in distilled water 10% (w/v) and 10% (v/v) respectively. This solution is stored in refrigerator at 4°C for a maximum time period of 6 months. The storage solution of the MEN1611 and the vehicle storage solution are diluted by 10 times with the diluent solution, by obtaining a dosage solution at 0.65 mg/ml.

*Trastuzumab* is a monoclonal antibody directed against the HER2 receptor used in the treatment of the HER2 positive classified breast cancer .

*Cetuximab* is a chimera (mouse/man) monoclonal antibody directed against the EGF receptor, used for the treatment of the colorectal metastatic, the non-small cell lung metastatic cancer and in the head and neck cancer.

*Bevacizumab* is a humanized recombinant monoclonal antibody which stops angiogenesis through the inhibition of the vascular and endothelial growth factor-A (VEGF-A), approved by FDA for the treatment of the metastatic colorectal cancer in combination with the standard chemotherapeutic treatment or as second place with 5-fluorouracil.

*Erlotinib* is an inhibitor of the epidermal growth factor receptor (EGFR). It is used in the treatment of advanced or metastatic non-small cell lung cancer (NSCLC), in the metastatic pancreatic cancer with or without mutations of the EGF receptor and even in other types of cancer.

Fulvestrant: a selective degrader/modulator of the estrogen receptor (ER). It is used in the treatment of the metastatic positive estrogen breast cancer, preferably in post-menopause patients.

Gefitinib is an inhibitor of the tyrosine kinase existing on the intracell side of the EGF receptor (EGFR). It is used in the treatment of the local advanced or metastatic non-small cell lung cancer (NSCLC) with mutations and over-expression of the EGF receptor.

### Xenograft tumour models

Females of 6-8-week-old athymic nude mice were obtained by Charles River (Calco, Lecco, Italy), kept in micro-isolating cages under continuous control of the environmental conditions. The drinking water and the specific food (VRF1, Charles River) were provided ad libitum. The environmental conditions, as well as the procedures for stabulation and manipulation of the animals were in conformity with the guidelines UKCCCR (4) and to the European Convention for the protection of the vertebrate animals used for experimental and scientific purposes (2010/63/EU; ref 5).

All xenograft tumour models were generated through a sub-cutaneous inoculum of 20 x 10⁶ cells, resuspended in 0.2 ml of BME type-3 5.6 mg/ml (TREVIGEN), on each right side of the mice.

The tumour growth was followed by measuring the length and the thickness of each tumour mass by using the gauge (twice a week). The tumour volume was calculated by using the following formula: Volume in mm³ = thickness² x length/2.

Even the body weight of mice was monitored. The treatments started when the tumour masses reached an average volume of 200-300 mm³.

Each group was formed by six/seven mice. Even the following effectiveness parameters were evaluated: - Inhibition of the tumour volume % (TVI%) in the treated mice compared to the control mice. - According to the directives for the protection of the vertebrate animals used for scientific purposes, the death caused by toxicity at the end of the study should be avoided and replaced by procedures which protect the animals. In particular, in our experiments, when the volume of the tumour masses reaches 10% of the total body weight of the mouse or when the body weight of the mice decreases more than 20% with respect to the control mice, for a period of at least 7 days, and accompanied even by absence of appetite, the mice are sacrificed by euthanasia with exposition to carbon dioxide, according to the standard procedures (Annex IV, in ref.5) . Examples 1 and 2 are reference examples.

### Example 1: Antitumour activity of MEN1611 in combination with trastuzumab in a JIMT-1 xenograft model of breast cancer HER2+, mutated on the PIK3CA gene.

We investigated the therapeutic potential of MEN1611 in combination with trastuzumab in two xenograft models of breast cancer HER2+, mutated in PIK3CA. In this study, we tested the effectiveness of MEN1611 at the clinically relevant dose of 6.5 mg/Kg by oral route, with an administration scheme of q1dx12, trastuzumab was used at a dose of 30 mg/Kg intraperitoneally, with an administration scheme of q7dx2.

The effectiveness was evaluated by using the % tumour volume decrease (%TVI).

In the JIMT-1 xenograft model, HER2+breast cancer, resistant to trastuzumab and bearing the mutation on PI3KCA p.C420R, MEN1611 in combination with trastuzumab showed a powerful synergic antitumour effect, in fact the animals of the group treated with combo, at the end of the study, obtained a decrease in the tumour volumes of 67.6% with respect to the control group. On the contrary, MEN1611 and trastuzumab in monotherapy determine -0.4% and 8.8% (Figure 1), respectively. No toxicity effects in terms of lowering of the body weights nor fatal events in all treated groups were observed.

Moreover, the activity of MEN1611 was even evaluated by means of its capability of inhibiting phosphorylation of two proteins downstream of the cascade of PI3K, AKT (Serina 473) signal and the S6 (240-244) factor, in the tumour masses of mice belonging to each group, collected after 4 hours of the third dose and last dose of MEN1611. Both phosphorylations were suppressed in the tumours of the mice treated with MEN1611 and with the combination of tratsuzumab, instead the levels of phosphorylation in the tumour masses of the mice treated with trastuzumab alone did not change. The constant levels of the two total proteins Akt and S6 showed that MEN1611 has no effects on the stability of these proteins (Figure 2).

The samples were analysed by immunoblotting with antibodies directed against phospohylated akt (pS473-Akt), total Akt, phosphorylation of S6 (240/244), and total S6 (Figure 2).

Example 2: *Antitumour activity of MEN1611 in combination with trastuzumab in a HCC1954 xenograft model resistant to trastuzumab, mutated on the PIK3CA gene.* The increase in the antitumour activity of MEN1611 in combination with trastuzumab was observed even in an additional xenograft model of HER2+ breast cancer, HCC1954, resistant to trastuzumab and bearing the mutation p.H1047R in the gene expressing PIK3.

The combination of the two molecules induces a decrease in the tumour volumes of 65.1%, evaluated at the end of the study. On the contrary, only 20.4% and 15.3% were induced in the mice treated with MEN1611 and trastuzumab, respectively (Figure 3). No toxic effects in terms of lowering of the body weight nor fatal events in all treated groups of mice were observed. Even in this model the pharmacodynamic of MEN1611 in combination with trastuzumab was confirmed (Figure 4).

The samples were analysed by immunoblotting with antibodies directed against phosporylated akt (pS473-Akt), total Akt, phosphorylation S6 (240/244), and total S6.

### Example 3: Antitumour activity of MEN1611 in combination with cetuximab in a HCC70 xenograft model of triple negative breast cancer (TNBC), wild type on the PIK3CA gene and with loss in PTEN gene.

The therapeutic potential of MEN1611 in combination with cetuximab was evaluated in a xenograft model of breast cancer TNBC wild type on the PIK3CA gene and with PTEN loss, which induces equally a hyper-activation of the pathway of PI3K. In this study, the effectiveness of MEN1611 was tested at the clinically relevant dose of 6.5 mg/kg by oral route, with a scheme of q1dx12, cetuximab was administered intraperitoneally with a dose of 30 mg/Kg, with an administration scheme of q7dx2. The combination of the two molecules, at the end of the study, determines a decrease in the tumour volumes of 60.2% with respect to the control group, on the contrary the 50.5% in the treated group with MEN1611 alone and 38.1% in the one treated with cetuximab (Figure 5). Even in this model the pharmacodynamics of MEN1611 in combination with cetuximab was confirmed (Figure 6).

### Example 4: Antitumour activity of MEN1611 in combination with cetuximab in a RKO xenograft model of colorectal cancer, mutated on PI3KCA, mutated on BRAF, but wild type (w.t.) for KRAS.

The therapeutic potential of MEN1611 in combination with cetuximab was evaluated in two xenograft models of colorectal cancer, mutated on PI3KCA, but w. t. for KRAS. In this study, the effectiveness of MEN1611 was tested with the clinically relevant dose of 6.5 mg/Kg by oral route with a treatment scheme q1dx12, cetuximab was used at a dose of 30 mg/Kg intraperitoneally, with an administration scheme q7dx2.

In the RKO xenograft model of colorectal cancer, bearing the mutation p.H1047R in PIK3CA, and pV600E in BRAF,MEN1611 in combination with Cetuximab shows a synergic antitumour effect, in fact at the end of the study, the group treated with the combination of the two drugs, has a decrease in the tumour volumes of 59.6%. On the contrary, MEN1611 and cetuximab alone induce 32.3% and 37.3%, respectively, of decrease in the tumour volumes (Figure 7).

No toxic effects in terms of lowering of body weight nor fatal events, in all treated groups, were observed. The phosphorylation of both proteins was suppressed in the tumour masses of the mice treated with MEN1611, on the contrary no variations in the tumour masses of the mice treated with cetuximab were observed. The constant levels of the two total proteins Akt and S6 designate that MEN1611 has no effects on the stability of these proteins (Figure 8). The samples were analysed by immunoblotting with antibodies directed against phosphorylated akt (pS473-Akt), total Akt, phosphorylation of S6 (240/244), and total S6.

*Example 5: Antitumour activity of MEN1611 in combination with cetuximab in a HT29 xenograft model of colorectal cancer, mutated on PIK3CA, mutated on BRAF, but w.t. for KRAS.* An effective antitumour activity of MEN1611 in combination with cetuximab, was also observed in an additional model of colorectal cancer, bearing the mutation p.P449T on the gene expressing PI3K . The combination of the two agents induces 89.4% of decrease in the tumour volumes, evaluated at Nadir (Figure 9). No toxic effects in terms of decrease in the body weight nor fatal events were observed. Even in this model, the pharmacodynamics of MEN1611 in combination with cetuximab was demonstrated (Figure 10). The samples were analysed by immunoblotting with antibodies directed against phosphorylated akt (pS473-Akt), total Akt, phosphorylation of S6 (240/244), and total S6.

### Example 6: Antitumour activity of MEN1611 in combination with bevacizumab in a PDX model of colorectal cancer, mutated on PI3KCA and KRAS.

The therapeutic potential of MEN1611 in combination with bevacizumab was evaluated in a PDX model of colorectal cancer, mutated on PIK3CA and KRAS. In this study, the effectiveness of MEN1611 was tested at the clinically relevant dose of 6.5 mg/Kg by oral route, with an administration scheme of q1dx12, bevacizumab was used at 2 mg/Kg intraperitoneally, with an administration scheme of 2 times a week for two weeks.

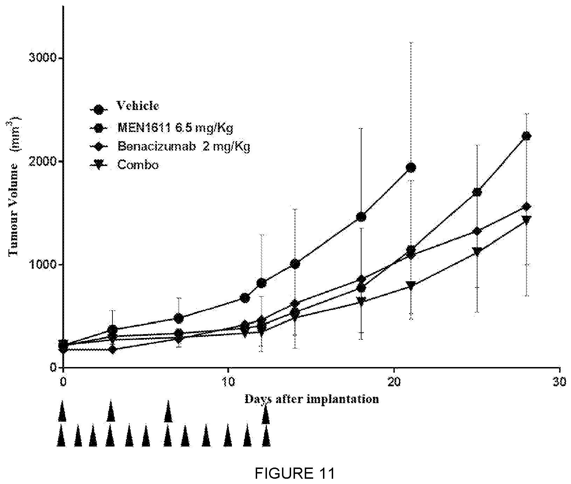

In the PDX CTG-1509 model of colorectal cancer, bearing the mutation p.H1047R on PIK3CA and p.Q61H on KRAS. MEN1611 in combination with bevacizumab showed a synergic effect by inducing a decrease of 67% in the tumour volumes. On the contrary, MEN1611 and bevacizumab alone induced only 46.1% and 46.9%, respectively (Figure 11). No toxic effects in terms of decrease in the body weights nor fatal events in all treatment groups were observed.

### Example 7: Antitumour activity of MEN1611 in combination with erlotinib in a NCI-H-1975 xenograft model of non-small cell lung cancer, mutated on the PIK3CA and EGFR genes.

The therapeutic potential of MEN1611 in combination with erlotinib, was evaluated in a xenograft model of non-small cell lung cancer, mutated on the PIK3CA and EGFR genes. In this study, the effectiveness of MEN1611 was tested at the clinically relevant dose of 6.5 mg/Kg by oral route, with a treatment scheme of q1dx12, erlotinib was tested at the dose of 50 mg/Kg by oral route, with a treatment scheme of q1dx6. A significant antitumour activity of the combination of MEN1611 with erlotinib was observed in the NCI-H-1975 xenograft model, with non-small cell lung cancer, bearing the mutation p.G118D in PIK3CA and the double mutation on the EGFR gene, p.T790M and p.L858R. The combination of the two molecules, at the end of the study induces a decrease in the tumour volumes of 51.4%. On the contrary MEN1611 and erlotinib alone do not induce decrease in the tumour volume. (Figure 12). Even in this model the pharmacodynamics of MEN1611 in combination with erlotinib was confirmed. (Figure 13). The samples were analysed by immunoblotting with antibodies directed against phosphorylated akt (pS473-Akt), total Akt, phosphorylation of S6 (240/244), and total S6.

### Example 8: Antitumour activity of MEN1611 in combination with erlotinib in a NCI-H-292 xenograft model of non-small cell lung cancer, w.t. for the PIK3CA and EGFR.

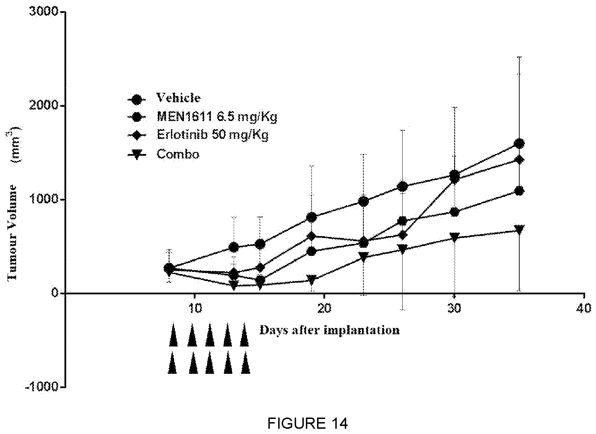

The therapeutic potential of MEN1611 in combination with erlotinib, was evaluated in a xenograft model of non-small cell lung cancer, w.t. for the PIK3CA and EGFR genes. In this study, the effectiveness of MEN1611 was tested at the clinically relevant dose of 6.5 mg/Kg by oral route, with a treatment scheme of q1dx5, erlotinib was tested at the dose of 50 mg/Kg by oral route, with a treatment scheme of q1dx5. A good antitumour activity of the combination of MEN1611 with erlotinib was observed in the NC-H-292 xenograft model of non-small cell lung cancer, w.t. for the PIK3CA and EGFR genes. The combination of the two molecules, evaluated at nadir, induces a decrease in the tumour volumes of 62% (Figure 14).

### Example 9: Synergic effect of the combination MEN1611 and Fulvestran on a cell line of breast cancer.

The T47D cell line of breast cancer HER2+/ER+, mutated on the PIK3CA gene was treated with increasing doses of MEN1611 (i.e. 0.013 - 0.068 - 0.34 - 1.7 - 8.5 uM) alone or in combination with fulvestrant (degrader/modulator of the estrogen receptor) (0.0049 - 0.024 - 0.124 - 0.62 uM). The Chou-Talalay combination index (CI- Chou-Talalay combination index CI) was estimated according to what designated in Preclinical versus Clinical Drugs Combination Studies. Chou TC. Leuk. Lymphoma. 5 2008;49(11):2059-2080).

Such index provides a quantitative definition of the synergy and in particular:
- CI <0.3 designates a strong synergy,
- 0.3 < CI <0.9 designates synergy,
- 0.9 < CI< 1.1 designates an additive effect,
- CI> 1.1 designates antagonism.

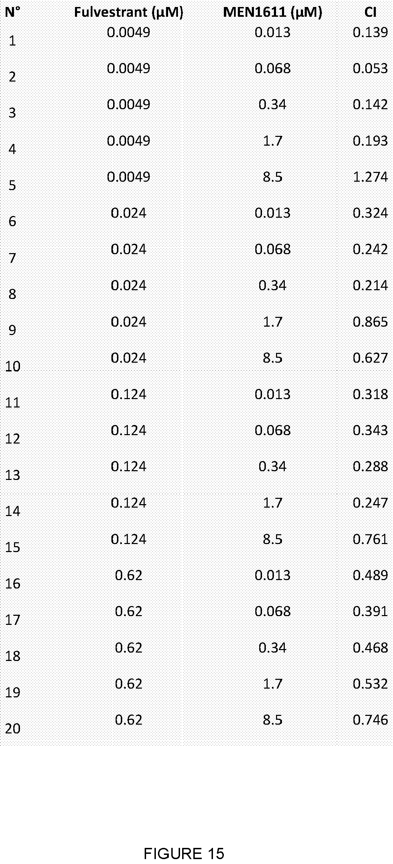

As shown in figure 15, the combination MEN161 and fulvestrant at different concentrations shows a CI value designating a strong synergy or synergy.

### Example 10: Antitumour activity of MEN1611 in combination with erlotinib in a PDX model of non-small cell lung cancer, mutated on the PIK3CA and EGFR genes.

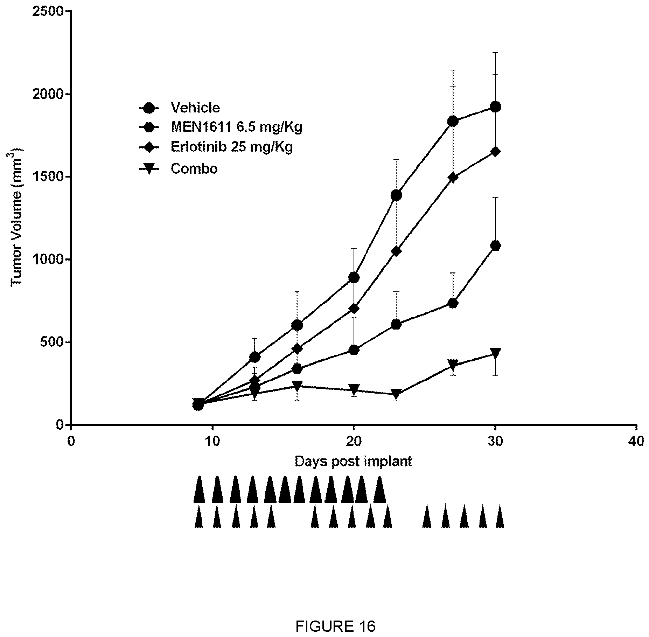

The therapeutic potential of MEN1611 in combination with erlotinib, was evaluated in a PDX model of non-small cell lung cancer, mutated on the PIK3CA and EGFR genes. In this study, the effectiveness of MEN1611 was tested at the clinically relevant dose of 6.5 mg/Kg by oral route, with a treatment scheme of q1dx12, erlotinib was tested at the dose of 25 mg/Kg by oral route, with a treatment scheme of q1dx5 for 3 weeks. A significant antitumour activity of the combination del MEN1611 with erlotinib was observed in the PDX RU278 model of non-small cell lung cancer, bearing the mutation p.E542K in PIK3CA and the deletion of exon 19 on the EGFR gene, p.E746_A750del. The combination of the two molecules, at the end of the study, induces a decrease in the tumour volumes of 77.7%. On the contrary MEN1611 and erlotinib alone induce a decrease in the tumour volume of 43.6% and 14% respectively (Figure 16).

### Example 11: Antitumour treatment of MEN1611 in combination with gefitinib in a RBI xenograft model of non-small cell lung cancer, mutated on the EGFR gene and wild type for the PIK3CA gene.

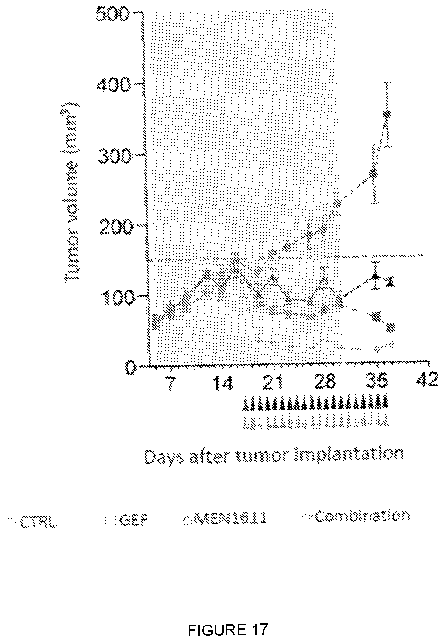

The therapeutic potential of MEN1611 in combination with gefitinib, was evaluated in a PDX model of non-small cell lung cancer, mutated on the EGFR gene and wild type for the PIK3CA gene. In this study, the effectiveness of MEN1611 was tested at the clinically relevant dose of 6.5 mg/Kg by oral route, with a treatment scheme of q1dx20, gefitinib was tested at the dose of 50 mg/Kg by oral route, with a treatment scheme of q1dx20. A significant antitumour activity of the combination of MEN1611 with gefitinib was observed in the RB1 model of non-small cell lung cancer, bearing the deletion of exon 19 on the EGFR gene, p.E746_A750del. The combination of the two molecules, at the end of the study induces a considerable decrease in the tumour volumes with respect to the MEN1611 and gefitinib alone (Figure 17).

## Claims

1. A pharmaceutical composition containing as active compound the PI3K-Class I inhibitor compound: 5-(7-methanesulfonyl-2-morpholin-4-yl-6,7-dihydro-5H-pyrrolo[2,3-d]pyrimidin-4-yl)-pyrimidin-2-amine or the pharmaceutically acceptable salt thereof, for use in an anticancer treatment in association with a second anticancer compound, and wherein said second anticancer compound is selected from the group comprising cetuximab, bevacizumab, fulvestrant, or gefitinib.

2. The pharmaceutical composition according to claim 1 for use in an anticancer treatment of colon cancer, prostate cancer, breast cancer, lung cancer, ovarian cancer.

3. The pharmaceutical composition according to claim 2 for use in the treatment of colorectal cancer with mutations of PI3KCA and *wild-type* KRAS, of colorectal cancer with mutations of PI3KCA and KRAS, of non-small cell lung carcinoma (NSCLC) with or without mutation on PI3KCA and EGFR, HER2-positive breast carcinoma with PI3KCA mutation, HER2-positive and ER-positive breast cancer.

4. The pharmaceutical composition for use according to any one of claims 1 to 3 suitable for the oral administration of the PI3k-Class I inhibitor, for use in association with a composition containing the second anticancer compound.

5. The pharmaceutical composition for use according to claim 4 comprising a quantity of PI3K-Class 1 inhibitor sufficient for a unitary dosage of 1 to 10 mg/Kg body weight.

6. The pharmaceutical composition for use according to claim 5 for a unitary dosage of the PI3K-Class 1 inhibitor from 5 to 7 mg/Kg body weight.

7. The pharmaceutical composition for use according to any one of claims 4 to 6 for use in association with a pharmaceutical composition suitable for the oral or parental administration of the second anticancer compound.

8. The pharmaceutical composition for use according to claim 7 for use in association with a pharmaceutical composition comprising a quantity of the second anticancer compound suitable for a unitary dosage of 10 to 80 mg/Kg body weight.

9. The pharmaceutical composition for use according to claim 8 for use in association with a pharmaceutical composition comprising a quantity of the second anticancer compound suitable for a unitary dosage of 20 to 70 mg/Kg body weight, preferably from 30 to 50 mg/Kg body weight.

10. Kit of parts comprising a pharmaceutical composition containing as active compound the PI3K-Class I inhibitor compound: 5-(7-methanesulfonyl-2-morpholin-4-yl-6,7-dihydro-5H-pyrrolo[2,3-d]pyrimidin-4-yl)-pyrimidin-2-amine or the pharmaceutically acceptable salt thereof, and a second pharmaceutical composition containing a second anticancer compound, and wherein said second anticancer compound is selected from the group comprising cetuximab, bevacizumab, fulvestrant, or gefitinib, for use in an anticancer combination treatment.

11. Kit of parts for use according to claim 10 wherein the anticancer combination treatment is colon, prostate, breast, lung, ovarian cancer.

12. Kit of parts according to claim 11 for use in a combination treatment of colorectal carcinoma with mutations of PI3KCA and *wild-type* KRAS, of colorectal carcinoma with mutations of PI3KCA and KRAS, of non-small cell lung carcinoma (NSCLC) with or without mutation on PI3KCA and EGFR, HER2-positive breast cancer with PI3KCA mutation, HER2-positive and ER-positive breast cancer.

13. Kit of parts for use according to any one of claims 10 to 12 wherein the first pharmaceutical composition comprises a quantity of PI3K-Class 1 inhibitor sufficient for a unitary dosage of 1 to 10 mg/Kg body weight, preferably from 5 to 7 mg/Kg body weight.

14. Kit of parts for use according to claim 13 wherein the second composition comprises a quantity of the second anticancer compound suitable for a unitary dosage of 10 to 80 mg/Kg body weight, preferably from 20 to 70 mg/Kg or from 30 to 50 mg/Kg body weight.

15. Kit of parts for use according to any one of claims 10 to 14 wherein the first composition is suitable for the oral administration of the PI3k-Class I inhibitor, and the second composition is suitable for the oral or parental administration.

## Patentansprüche

1. Pharmazeutisches Stoffgemisch, enthaltend als Wirkstoff die PI3K-Klasse-I-Inhibitorverbindung: 5-(7-Methansulfonyl-2-morpholin-4-yl-6,7-dihydro-5H-Pyrrolo[2,3-d]pyrimidin-4-yl)-pyrimidin-2-amin oder das pharmazeutisch annehmbare Salz davon, zur Verwendung bei einer Antikrebsbehandlung in Verbindung mit einem zweiten Antikrebsstoffgemisch, und wobei das zweite Antikrebsstoffgemisch aus der Gruppe ausgewählt ist, die Cetuximab, Bevacizumab, Fulvestrant oder Gefitinib umfasst.

2. Pharmazeutisches Stoffgemisch gemäß Anspruch 1 zur Verwendung bei einer Antikrebsbehandlung von Darmkrebs, Prostatakrebs, Brustkrebs, Lungenkrebs, Eierstockkrebs.

3. Pharmazeutisches Stoffgemisch gemäß Anspruch 2 zur Verwendung bei der Behandlung von Dickdarmkrebs mit PI3KCA- und *Wildtyp*-KRAS-Mutationen, von Dickdarmkrebs mit P13KCA- und KRAS-Mutationen, von nicht-kleinzelligem Lungenkarzinom (NSCLC) mit oder ohne PI3KCA- und EGFR-Mutation, HER2-positivem Brustkarzinom mit PI3KCA-Mutation, HER2-positivem und ER-positivem Brustkrebs.

4. Pharmazeutisches Stoffgemisch zur Verwendung gemäß einem der Ansprüche 1 bis 3, das für die orale Verabreichung des PI3k-Klasse-I-Inhibitors geeignet ist, zur Verwendung in Verbindung mit einem Stoffgemisch, das das zweite Antikrebsstoffgemisch umfasst.

5. Pharmazeutisches Stoffgemisch zur Verwendung gemäß Anspruch 4, umfassend eine Menge an PI3K-Klasse-1-Inhibitor, die für eine Einheitsdosis von 1 bis 10 mg/Kg Körpergewicht ausreicht.

6. Pharmazeutisches Stoffgemisch zur Verwendung gemäß Anspruch 5 für eine Einheitsdosis des PI3K-Klasse-1-Inhibitors von 5 bis 7 mg/Kg Körpergewicht.

7. Pharmazeutisches Stoffgemisch zur Verwendung gemäß einem der Ansprüche 4 bis 6 zur Verwendung in Verbindung mit einem pharmazeutischen Stoffgemisch, das für die orale oder parenterale Verabreichung des zweiten Stoffgemisches geeignet ist.

8. Pharmazeutisches Stoffgemisch zur Verwendung gemäß Anspruch 7 zur Verwendung in Verbindung mit einem pharmazeutischen Stoffgemisch, das eine Menge des zweiten Stoffgemisches umfasst, die für eine Einheitsdosis von 10 bis 80 mg/Kg Körpergewicht geeignet ist.

9. Pharmazeutisches Stoffgemisch zur Verwendung gemäß Anspruch 8 zur Verwendung in Verbindung mit einem pharmazeutischen Stoffgemisch, das eine Menge des zweiten Antikrebsstoffgemischs umfasst, die für eine Einheitsdosierung von 20 bis 70 mg/Kg Körpergewicht, vorzugsweise von 30 bis 50 mg/Kg Körpergewicht, geeignet ist.

10. Kit aus Teilen, umfassend ein pharmazeutisches Stoffgemisch, das als aktiven Wirkstoff die PI3K-Klasse-I-Inhibitorverbindung enthält: 5-(7-Methansulfonyl-2-morpholin-4-yl-6,7-dihydro-5H-pyrrolo[2,3-d]pyrimidin-4-yl)-pyrimidin-2-amin oder das pharmazeutisch annehmbare Salz davon, und ein zweites pharmazeutisches Stoffgemisch, das eine zweite Antikrebsverbindung enthält, und wobei die zweite Antikrebsverbindung ausgewählt ist aus der Gruppe, die Cetuximab, Bevacizumab, Fulvestrant oder Gefitinib umfasst, zur Verwendung bei einer Antikrebs-Kombinationsbehandlung.

11. Kit aus Teilen zur Verwendung gemäß Anspruch 10, wobei die Antikrebs-Kombinationsbehandlung Darm-, Prostata-, Brust-, Lungen- oder Eierstockkrebs ist.

12. Kit aus Teilen gemäß Anspruch 11 zur Verwendung in einer Kombinationsbehandlung von Dickdarmkrebs mit PI3KCA- und *Wildtyp*-KRAS-Mutationen, von Dickdarmkrebs mit PI3KCA- und KRAS-Mutationen, von nicht-kleinzelligem Lungenkarzinom (NSCLC) mit oder ohne PI3KCA- und EGFR-Mutation, HER2-positivem Brustkrebs mit PI3BKCA-Mutation, HER2-positivem und ER-positivem Brustkrebs.

13. Kit aus Teilen zur Verwendung gemäß einem der Ansprüche 10 bis 12, wobei das erste pharmazeutische Stoffgemisch eine Menge an PI3K-Klasse-1-Inhibitor umfasst, die für eine Einheitsdosis von 1 bis 10 mg/Kg Körpergewicht, vorzugsweise von 5 bis 7 mg/Kg Körpergewicht, ausreicht.

14. Kit aus Teilen zur Verwendung gemäß Anspruch 13, wobei das zweite Stoffgemisch eine Menge des zweiten Antikrebsstoffgemischs umfasst, die für eine Einheitsdosierung von 10 bis 80 mg/Kg Körpergewicht, vorzugsweise von 20 bis 70 mg/Kg oder von 30 bis 50 mg/Kg Körpergewicht, geeignet ist.

15. Kit aus Teilen zur Verwendung gemäß einem der Ansprüche 10 bis 14, wobei das erste Stoffgemisch für die orale Verabreichung des PI3k-Klasse-I-Inhibitors und das zweite Stoffgemisch für die orale oder parenterale Verabreichung geeignet ist.

## Revendications

1. Composition pharmaceutique contenant, en tant que principe actif, le composé inhibiteur de PI3K de classe I : 5-(7-méthanesulfonyl-2-morpholin-4-yl-6,7-dihydro-5H-pyrrolo[2,3-d]pyrimidin-4-yl)pyrimidin-2-amine ou son sel pharmaceutiquement acceptable, pour une utilisation dans un traitement anticancéreux en association avec un deuxième composé anticancéreux, et dans laquelle le deuxième composé anticancéreux est choisi dans le groupe comprenant le cétuximab, le bévacizumab, le fulvestrant, et le géfitinib.

2. Composition pharmaceutique selon la revendication 1, pour une utilisation dans un traitement anticancéreux d'un cancer du côlon, d'un cancer de la prostate, d'un cancer du sein, d'un cancer du poumon, d'un cancer ovarien.

3. Composition pharmaceutique selon la revendication 2, pour une utilisation dans le traitement d'un cancer colorectal avec des mutations de PI3KCA et de KRAS de type sauvage, d'un cancer colorectal avec des mutations de PI3KC3 et de KRAS, d'un carcinome pulmonaire non à petites cellules (NSCLC) avec ou sans mutation sur PI3KCA et EGFR, d'un carcinome mammaire HER2-positif avec une mutation de PI3KCA, d'un cancer du sein HER2-positif et ER-positif.

4. Composition pharmaceutique pour une utilisation selon l'une quelconque des revendications 1 à 3, convenant pour l'administration par voie orale de l'inhibiteur de PI3k de classe I, pour une utilisation en association avec une composition contenant le deuxième composé anticancéreux.

5. Composition pharmaceutique pour une utilisation selon la revendication 4, comprenant une quantité d'inhibiteur de PI3K de classe 1 suffisante pour une dose unitaire de 1 à 10 mg/kg de poids corporel.

6. Composition pharmaceutique pour une utilisation selon la revendication 5, pour une dose unitaire de l'inhibiteur de PI3K de classe 1 de 5 à 7 mg/kg de poids corporel.

7. Composition pharmaceutique pour une utilisation selon l'une quelconque des revendications 4 à 6, pour une utilisation en association avec une composition pharmaceutique contenant pour l'administration par voie orale ou parentérale du deuxième composé anticancéreux.

8. Composition pharmaceutique pour une utilisation selon la revendication 7, pour une utilisation en association avec une composition pharmaceutique comprenant une quantité du deuxième composé anticancéreux convenant pour une dose unitaire de 10 à 80 mg/kg de poids corporel.

9. Composition pharmaceutique pour une utilisation selon la revendication 8, pour une utilisation en association avec une composition pharmaceutique comprenant une quantité du deuxième composé anticancéreux convenant pour une dose unitaire de 20 à 70 mg/kg de poids corporel, de préférence de 30 à 50 mg/kg de poids corporel.

10. Ensemble de substances comprenant une composition pharmaceutique contenant, en tant que principe actif, le composé inhibiteur de PI3K de classe I : 5-(7-méthanesulfonyl-2-morpholin-4-yl-6,7-dihydro-5H-pyrrolo[2,3-d]pyrimidin-4-yl)pyrimidin-2-amine ou son sel pharmaceutiquement acceptable, et une deuxième composition pharmaceutique contenant un deuxième composé anticancéreux, et dans lequel ledit deuxième composé anticancéreux est choisi dans le groupe comprenant le cétuximab, le bévacizumab, le fulvestrant, et le géfitinib, pour une utilisation dans un traitement anticancéreux combiné.

11. Ensemble de substances pour une utilisation selon la revendication 10, dans lequel le traitement anticancéreux combiné est pour un cancer du côlon, de la prostate, du sein, du poumon, ovarien.

12. Ensemble de substances selon la revendication 11 pour une utilisation dans un traitement combiné d'un carcinome colorectal avec des mutations de PI3KCA et de KRAS de type sauvage, d'un carcinome colorectal avec des mutations de PI3KC3 et de KRAS, d'un carcinome pulmonaire non à petites cellules (NSCLC) avec ou sans mutation sur PI3KCA et EGFR, d'un cancer du sein HER2-positif avec une mutation de PI3KCA, d'un cancer du sein HER2-positif et ER-positif.

13. Ensemble de substances pour une utilisation selon l'une quelconque des revendications 10 à 12, dans lequel la première composition pharmaceutique comprend une quantité d'inhibiteur de PI3K de classe 1 suffisante pour une dose unitaire de 1 à 10 mg/kg de poids corporel, de préférence de 5 à 7 mg/kg de poids corporel.

14. Ensemble de substances pour une utilisation selon la revendication 13, dans lequel la deuxième composition comprend une quantité du deuxième composé anticancéreux convenant pour une dose unitaire de 10 à 80 mg/kg de poids corporel, de préférence de 20 à 70 mg/kg de poids corporel ou de 30 à 50 mg/kg de poids corporel.

15. Ensemble de substances pour une utilisation selon l'une quelconque des revendications 10 à 14, dans lequel la première composition convient pour l'administration par voie orale de l'inhibiteur de PI3k de classe I, et la deuxième composition convient pour une administration par voie orale ou parentérale.
